# EUROPÄISCHE PATENTSCHRIFT

(11) **EP 3 200 860 B1**
(45) Veröffentlichungstag und Bekanntmachung des Hinweises auf die Patenterteilung: **13.01.2021**
(21) Anmeldenummer: 14793025.9
(22) Anmeldetag: 02.10.2014
(51) Int. Cl.: A61M 25/10, B29C 63/00, B29C 63/18

(54) **CUT BACK VERFAHREN FÜR INTRAVASKULÄRE DILATATIONSKATHETER**
CUTBACK METHOD FOR INTRAVASCULAR DILATION CATHETER
PROCÉDÉ DÉCOUPE DE CATHÉTER À DILATATION INTRA-VASCULAIRE

(43) Veröffentlichungstag der Anmeldung: 09.08.2017
(73) Patentinhaber: Rübben, Alexander, 98000 Monaco (MC); Aachen Scientific International Pte. Ltd., Singapore 079903 (SG)
(72) Erfinder: RÜBBEN, Alexander, 98000 Monaco (MC)
(74) Vertreter: Schneiders, Josef
(86) Internationale Anmeldenummer: PCT/EP2014/071114
(87) Internationale Veröffentlichungsnummer: WO 2016/050303

(56) Entgegenhaltungen:
- EP-A1- 1 925 332
- US-A1- 2002 099 431
- US-A1- 2005 251 107
- US-B1- 6 613 067
- US-B1- 7 147 817

## Beschreibung

Die Erfindung betrifft ein Verfahren zur Herstellung eines Katheters mit einem proximalen Abschnitt, einem distalen Abschnitt und einem im distalen Abschnitt angeordneten expandierbaren Element, wobei das expandierbare Element durch Zufuhr eines Fluids aus einem kontrahierten Zustand in einen expandierten Zustand bringbar und eine entfernbare Schutzeinrichtung in Form eines Schlauchs um das expandierbare Element im kontrahierten Zustand angeordnet ist.

Bei der perkutanen transluminalen coronaren Angioplastie (PTCA) wird ein Dilatationsballonkatheter (Dilatationskatheter, Ballonkatheter) verwendet, der in seinem distalen Abschnitt über ein expandierbares Element (Ballon) verfügt. Der Zugang zum Kardiovaskulärsystem des Patienten erfolgt über ein Einführgerät, meist in der Leistengegend über die Arteria femoralis. Die einzelnen medizinischen Vorrichtungen werden über das Einführgerät perkutan in das Kardiovaskulärsystem des Patienten eingeführt und vorgeschoben, bis sich das distale Ende an der gewünschten Position befindet. Im Einzelnen wird ein Führungskatheter eingeführt, durch den ein Führungsdraht sowie der Ballonkatheter vorgeschoben werden. Der Führungsdraht wird zuerst aus dem Führungskatheter vorgeschoben, anschließend wird der Ballonkatheter, der über ein Lumen zur Aufnahme des Führungsdrahts verfügt, über den Führungsdraht so weit nach distal bewegt, bis sich der Ballon an der Stelle der Läsion, d. h. der zu beseitigenden Gefäßverengung (Stenose) befindet. Der Ballonkatheter verfügt über ein Zufuhrlumen, das sich durch den Ballonkatheter bis proximal erstreckt und am distalen Ende mit dem Ballon in Verbindung steht. Durch Zufuhr eines Fluids wird der Ballon mit hohem Druck von in der Regel mindestens 4 bar, häufig 8 bis 12 bar, expandiert, so dass Ablagerungen im Bereich der Läsion gegen die Innenwand der Arterie gepresst werden, um auf diese Weise die Stenose zu beseitigen und den Blutdurchfluss zu verbessern. Anschließend wird der Ballon zu einem kleinen Querschnitt zusammengelegt, so dass der Ballonkatheter aus dem Gefäßsystem des Patienten entfernt werden kann und der Blutfluss durch die geweitete Arterie wieder einsetzt.

Ballonkatheter sind beispielsweise aus den nachfolgenden US-Patentschriften bekannt: US 4,762,129, US 5,496,346 und US 6,071,285.

US 6 613 067 B1 beschreibt ein Verfahren zur Herstellung eines Katheters gemäß dem Oberbegriff des Anspruchs 1.

Medizinische Vorrichtungen mit einem Katheter und einem im distalen Abschnitt angeordneten expandierbaren Element werden auch zur Aufweitung von Stents verwendet. Hier steht nicht das Pressen von Ablagerungen an die Arterienwand, sondern die radiale Expansion des Stents im Vordergrund.

Ein wichtiges Merkmal eines Ballonkatheters ist sein Querschnitt, der durch den Außendurchmesser des distalen Abschnitts des Ballons im nicht expandierten Zustand bestimmt ist. Durch den Außendurchmesser wird beeinflusst, wie gut sich der Ballonkatheter durch die Koronararterien und über eine dichte Läsion an den Zielort bringen lässt, wobei ein möglichst kleiner Außendurchmesser wünschenswert ist. Gleichwohl muss die Möglichkeit erhalten bleiben, den Ballon am Zielort auf die gewünschten Dimensionen zu expandieren. Die US-Patente 5,342,307 und 5,015,231 offenbaren in diesem Zusammenhang eine Ballonschutzhülse um einen mindestens dreifach gefalteten Ballon eines Ballonkatheters für die Angioplastie.

Die Verkleinerung des Durchmessers des expandierbaren Teils des Ballonkatheters ("Cut Back") ist für Ballonkatheter und Systeme zur Implantierung von Stents wichtig, weil bestimmte Gefäßengstellen (Stenosen) für Ballonkatheter mit normalem Durchmesser nicht passierbar sind. Es stellt sich daher die Aufgabe, einen Katheter zur Verfügung zu stellen, dessen Querschnitt im distalen Abschnitt, in dem sich das expandierbare Element befindet, minimiert ist. Des Weiteren stellt sich die Aufgabe, ein Verfahren zur Herstellung eines entsprechenden Katheters zur Verfügung zu stellen.

Diese Aufgabe wird erfindungsgemäß gelöst durch ein Verfahren zur Herstellung eines Katheters mit einem proximalen Abschnitt, einem distalen Abschnitt und einem im distalen Abschnitt angeordneten expandierbaren Element, wobei das expandierbare Element durch Zufuhr eines Fluids aus einem kontrahierten Zustand in einen expandierten Zustand bringbar und eine entfernbare Schutzeinrichtung in Form eines Schlauchs um das expandierbare Element im kontrahierten Zustand angeordnet ist, wobei der Schlauch über das expandierbare Element gestülpt und mit dem Katheter durch eine oder mehrere Düsen geführt wird, wobei der Schlauch gestreckt wird und sich der Innendurchmesser des Schlauchs verkleinert.

Erfindungsgemäß wird ein Schlauch über das expandierbare Element (Ballon) gestülpt und zusammen mit dem Katheter (Ballonkatheter) durch eine sehr enge Düse geführt. Insbesondere kann der Katheter mit dem Schlauch durch die Düse gezogen werden. Auf diese Weise wird der Schlauch gestreckt und legt sich eng an das expandierbare Element an, so dass der Außendurchmesser des Katheters im distalen Abschnitt stark verringert wird. Der Schlauch bildet somit eine Hülle um das expandierbare Element. Das Ballonmaterial des expandierbaren Elements wird so eng ineinander gelegt, dass der Durchmesser insgesamt nur noch durch die Dicke der verwendeten Materialien bestimmt wird und nicht durch evtl. Lufteinschlüsse oder Spannungen, die den Ballon wieder aufstellen. In vielen Fällen wird das expandierbare Element bereits vor dem Überziehen des Schlauchs in Falten gelegt, um einen möglichst geringen Querschnitt herbeizuführen. Durch das Überziehen des Schlauchs wird eine zusätzliche Komprimierung und Querschnittsverringerung herbeigeführt. Typischerweise lässt sich eine Reduzierung des Durchmessers auf 4 French (1,33 mm) oder weniger herbeiführen.

Häufig ist es sinnvoll, den Ballonkatheter mit Schlauch nacheinander durch Düsen unterschiedlichen Durchmessers zu führen, wobei der Innendurchmesser von Düse zu Düse abnimmt. Der Katheter mit Schlauch wird somit zunächst auf einen ersten reduzierten Durchmesser gebracht, bevor mittels Durchführung durch eine Düse mit noch kleinerem Durchmesser ein zweiter, weiter reduzierter Durchmesser erreicht wird. Dies lässt sich grundsätzlich wiederholen, um den Durchmesser noch weiter zu verringern, wobei typischerweise nicht mehr als zwei bis vier Durchführungen durch Düsen mit jeweils geringerem Innendurchmesser in Betracht kommen.

Der Katheter kann einen inneren Tubus oder Schaft aufweisen, der sich typischerweise durch das Innere des Katheters über einen Teil der Länge oder die gesamte Länge erstreckt. Der innere Tubus weist ein Lumen zur Aufnahme eines Führungsdrahts auf, welches am distalen Ende des Katheters endet, sodass der Führungsdraht am distalen Ende austreten kann. Grundsätzlich sind verschiedene Ballonkatheter bekannt, insbesondere wird zwischen der Overthe-Wire-Technik (OTW-Katheter) und dem Rapid Exchange (Rx-Katheter) unterschieden. Während beim OTW-Katheter sich der Führungsdraht durch das Gesamtvolumen hindurch erstreckt, weisen Rx-Katheter eine Durchtrittsöffnung als Zugang zum inneren Tubus auf, durch die der Führungsdraht proximal des Ballons, aber distal des proximalen Ballonkatheterabschnitts austreten kann, bei dem es sich häufig um einen metallischen Hypotube handelt. Ebenfalls über die Länge des Ballonkatheters erstreckt sich ein Zufuhrlumen, durch das ein Fluid zum eigentlichen Ballon geführt werden kann, um diesen am Zielort aufzuweiten. Dieses Zufuhrlumen kann z. B. um den inneren Tubus herum oder seitlich davon angeordnet sein.

Die Düse kann grundsätzlich unterschiedliche Formen aufweisen. Wichtig ist, dass der Querschnitt der Düse so gewählt wird, dass der Schlauch eng auf das expandierbare Element aufbringbar ist. Beispielsweise kann die Düse zylinderförmig sein, d.h. die Form eines Hohlzylinders aufweisen, wobei die Grundfläche des Zylinders ein Kreis oder eine Ellipse sein kann. Insbesondere kann es sich um einen geraden Zylinder handeln, denkbar ist jedoch grundsätzlich auch ein schiefer Zylinder.

Als Düse wird aber erfindungsgemäß grundsätzlich eine beliebige Öffnung mit kleinem Innendurchmesser verstanden, die in der Lage ist, Katheter und Schlauch auf ein gewünschtes Maß zu komprimieren. Die Länge der Düse kann unterschiedlich sein; eine bestimmte Länge ist nicht erforderlich, auch wenige Millimeter können ausreichend sein. Praktisch kann man in der Weise vorgehen, dass in einem bestimmten Werkzeug mehrere Öffnungen vorgesehen sind, die unterschiedliche Durchmesser aufweisen, so dass beim Durchziehen von Katheter und Schlauch durch unterschiedliche Öffnungen der Durchmesser immer weiter abnimmt. Das Durchziehen kann manuell oder maschinell erfolgen.

Die Düse kann auch eine Konusform aufweisen, d.h. das Innere der Düse kann die Form eines Kegels oder eines Kegelstumpfes haben. Sinnvoll ist insbesondere eine sich vom Eintrittsende zum Austrittsende verjüngende Düse, um das im Eingangsbereich noch relativ weite expandierbare Element auf den Enddurchmesser zu bringen.

Um den Vorgang weiter zu verbessern, kann die Düse beim Durchziehen des Schlauchs erwärmt werden. Nach Abkühlen zieht sich der Schlauch noch etwas weiter zusammen und sorgt auf diese Weise für eine weitere Verringerung des Querschnitts des expandierbaren Elements. Die Erwärmung erfolgt auf eine Temperatur über Raumtemperatur, insbesondere auf eine Temperatur zwischen 40 und 100 °C, insbesondere zwischen 50 und 80 °C.

Sinnvoll ist es darüber hinaus, vor dem Überstülpen des Schlauchs über den distalen Abschnitt evtl. vorhandene Luft aus dem expandierbaren Element herauszusaugen, sodass diese nicht erst beim Komprimierungsprozess selbst herausgepresst werden muss. Weiterhin ist es von Vorteil, wenn das expandierbare Element vor dem Überziehen des Schlauchs bereits in Falten gelegt ist, wobei sich 2 bis 4, insbesondere 3 Falten besonders bewährt haben. Das Infaltenlegen kann manuell oder maschinell erfolgen.

Als Schlauch wird erfindungsgemäß eine Hülle aus einem verformbaren Material verstanden, die sich über einen Teil des Katheters ziehen lässt, insbesondere den Bereich des distalen Abschnitts, in dem sich das expandierbare Element befindet. Der Schlauch ist zumindest einseitig offen, um ein Überziehen über den Katheter zu ermöglichen, kann aber auch an beiden Enden offen sein.

Der Schlauch sollte aus einem Material gefertigt sein, das eine geringe Reibung erzeugt, damit das Material des darunter liegenden expandierbaren Elements nicht geschädigt wird. Besonders geeignet ist Polytetrafluorethylen (PTFE, Teflon). Möglich ist jedoch auch die Verwendung anderer Materialien, insbesondere Kunststoffmaterialien, die eine geringe Reibung aufweisen.

Unmittelbar vor Gebrauch des Katheters muss der Schlauch abgezogen werden. Dabei hat sich herausgestellt, dass das expandierbare Element durch Aufziehen des Schlauchs dauerhaft seinen Querschnitt verringert, d.h. das expandierbare Element bleibt eng zusammengefaltet mit minimalem Durchmesser, bis das Element am Einsatzort durch Zufuhr eines Fluids unter hohem Druck expandiert wird.

Besondere Bedeutung hat die Erfindung für mit Wirkstoff beladene Ballons, auch Drug Eluting Balloons genannt. Diese weisen meist einen größeren Querschnitt auf als unbeladene Ballons, z. B. weil sich zwischen den Falten, in die der Ballon im kontrahierten Zustand gelegt wird, ein Wirkstoff wie Paclitaxel befindet. Insbesondere bei sehr langen Ballons macht dies das Vorschieben im Blutgefäßsystem schwierig.

Ein durch das erfindungsgemäße Verfahren I herstellbare Katheter umfasst einen proximalen Abschnitt, einen distalen Abschnitt und ein im distalen Abschnitt angeordnetes expandierbares Element, wobei das expandierbare Element durch Zufuhr eines Fluids aus einem kontrahierten Zustand in einen expandierten Zustand bringbar ist. Darüber hinaus umfasst der Katheter eine entfernbare Schutzeinrichtung in Form eines Schlauchs, der um das expandierbare Element im kontrahierten Zustand angeordnet ist, wobei der Schlauch über das expandierbare Element gestülpt und in der Länge gestreckt ist. Durch den Katheter verläuft in Längsrichtung bis nach proximal ein Zufuhrlumen, das am distalen Ende mit dem expandierbaren Element in Verbindung steht und der Zufuhr eines Fluids zur Expansion des expandierbaren Elements dient. Darüber hinaus kann der Katheter einen inneren Tubus zur Aufnahme eines Führungsdrahts aufweisen.

Der proximale und der distale Abschnitt des Katheters können aus unterschiedlichen Materialien gefertigt sein, um den Ballonkatheter optimal einsetzen zu können. Während für den distalen Abschnitt eine große Flexibilität von Vorteil ist, damit der Katheter auch englumigen Blutgefäßen problemlos folgen kann, ohne diese zu verletzen, ist im proximalen Bereich eine höhere Steifigkeit von Vorteil, damit der Katheter über längere Distanzen vorschiebbar ist. Entsprechend kann der proximale Abschnitt aus Metall, z. B. aus Edelstahl gefertigt werden, während sich für den distalen Abschnitt Polymermaterialien wie Polyamide, beispielsweise Nylon, bewährt haben. Grundsätzlich sind aber auch Ballonkatheter herstellbar, bei denen proximaler und distaler Abschnitt aus dem gleichen Material gefertigt sind bzw. bei denen sich proximaler und distaler Abschnitt hinsichtlich des Aufbaus letztlich nicht unterscheiden und entsprechend kein Übergang zwischen den Abschnitten auszumachen ist.

Die Erfindung wird auch in den beigefügten Figuren näher erläutert.

Es zeigen:
- Figur 1: einen erfindungsgemäßen Katheter in der Seitenansicht;
- Figur 2: einen erfindungsgemäßen Katheter ohne Schlauch im Querschnitt;
- Figur 3: einen Schlauch im Querschnitt;
- Figur 4: einen erfindungsgemäßen Katheter mit Schlauch im Querschnitt und
- Figur 5: das Durchführen von Ballon und Schlauch durch eine Düse im Längsschnitt.

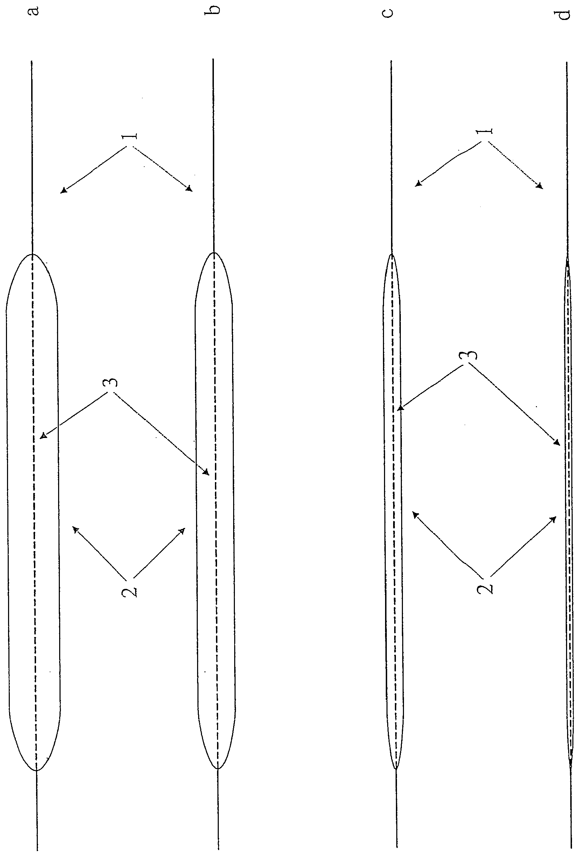

In Figur 1 sind verschiedene Darstellungen des distalen Bereichs eines Ballonkatheters 1 gemäß der Erfindung gezeigt. Der Ballonkatheter 1 verfügt in seinem distalen Endabschnitt über einen Ballon 2. Darüber hinaus verfügt der Ballonkatheter 1 über einen inneren Tubus 9, der auch durch das Innere des Ballons 2 verläuft.

In Figur 1a ist der Ballon 2 im expandierten Zustand zu erkennen, nachdem er durch Zufuhr eines Fluids unter Druck expandiert wurde. In dieser Form ist der Ballon 2 geeignet, Stenosen zu beseitigen.

In Figur 1b ist der Ballon 2 im nicht expandierten Zustand zu erkennen. Der Querschnitt des Ballons 2 ist zwar geringer als gemäß Figur 1a, jedoch zu groß, um durch englumige Blutgefäße geführt zu werden.

In Figur 1c ist der Ballon 2 im gefalteten Zustand dargestellt, so wie es dem Stand der Technik entspricht. Die nicht expandierte Ballonhülle wird dabei in einer Weise zusammengelegt, dass sie sich eng anlegt, so dass der Querschnitt gegenüber Figur 1b signifikant verringert ist.

In Figur 1d schließlich ist der Ballon 2 nach Anwendung des erfindungsgemäßen Verfahrens dargestellt. Durch Überziehen eines eng anliegenden Schlauchs sowie Durchführen durch eine Düse wird dafür gesorgt, dass sich der Querschnitt des Ballons 2 weiter verringert und der Katheter 1 im Bereich des Ballons 2 nur noch einen geringfügig größeren Durchmesser hat als in weiter proximal liegenden Bereichen. Diesen geringen Querschnitt behält der Ballon 2 auch bei, wenn kurz vor Gebrauch der Schlauch abgezogen wird.

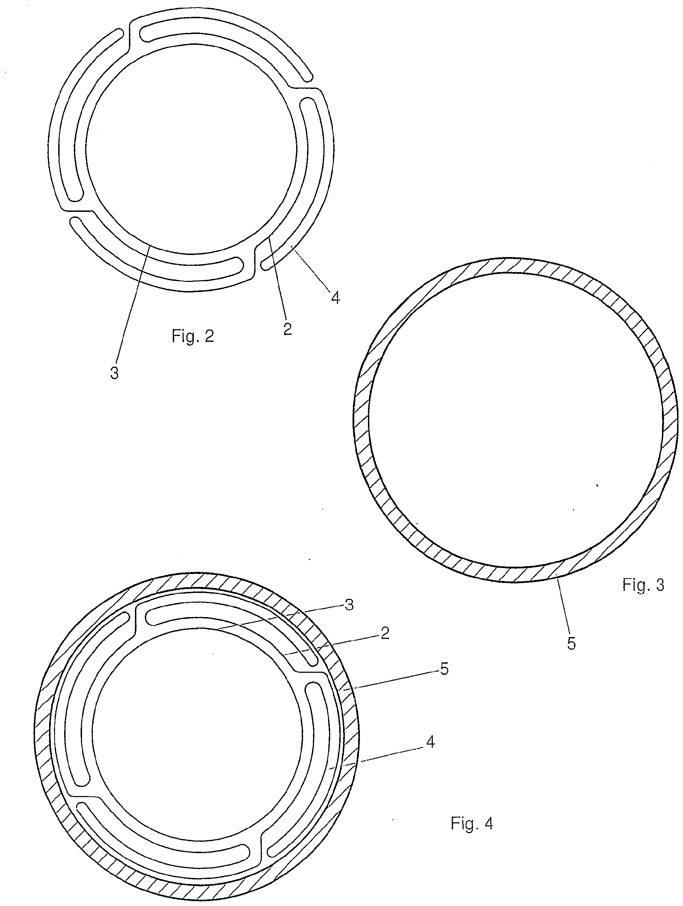

Figur 2 ist eine Darstellung des Ballonkatheters 1 ohne Schlauch im Querschnitt. Der Ballon 2 bildet hier drei Falten 4, die entlang des Umfangs in der gleichen Umfangsrichtung gelegt sind, um auf diese Weise für ein enges Anlegen des Ballons 2 und eine Verringerung des Durchmessers zu sorgen. Durch das Innere des Ballons verläuft der zum Ballonkatheter 1 gehörige innere Tubus 3.

In Figur 3 ist ein Schlauch 5 dargestellt, wie er verwendet werden kann, um über den Ballonkatheter 1 gezogen zu werden. Der Schlauch 5 ist aus einem Material gefertigt, das möglichst wenig Reibung mit der Oberfläche des Ballons 2 erzeugt, insbesondere aus Polytetrafluorethylen.

Figur 4 zeigt den Ballonkatheter 1, der mit dem Schlauch 5 überzogen wurde, indem Ballonkatheter 1 und Schlauch 5 erfindungsgemäß gemeinsam durch eine Düse gezogen wurden. Der Schlauch 5 legt sich eng an den Ballon 2 an und drückt diesen noch mehr zusammen, so dass sich der Querschnitt weiter verringert. Während die Figur 4 der besseren Erkennbarkeit wegen noch einen gewissen Abstand zwischen dem Schlauch 5, den einzelnen Falten 4 und den weiteren Bereichen des Ballons 2 zeigt, sind diese in der Realität nicht vorhanden, d. h. sämtliche Lufteinschlüsse sind praktisch vollständig entfernt, so dass der Querschnitt insgesamt minimal ist.

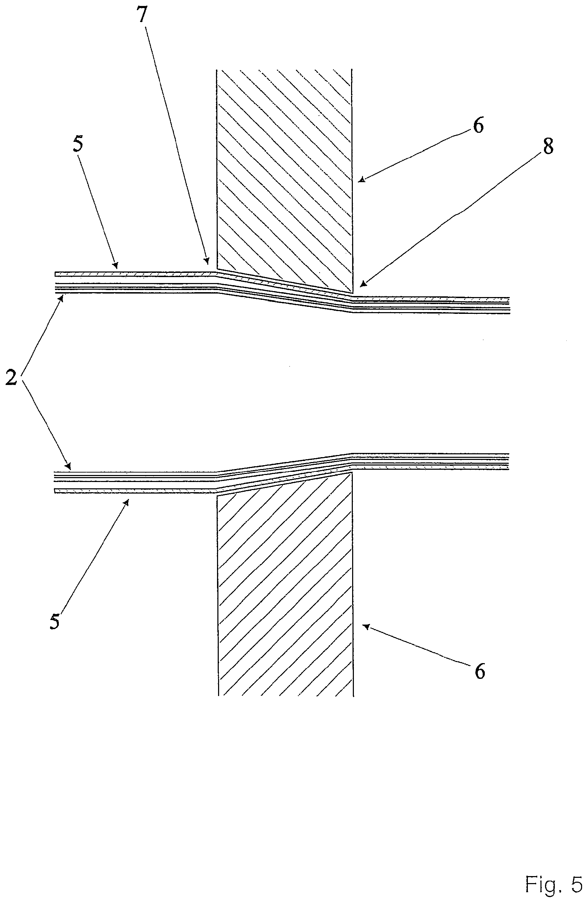

In Figur 5 schließlich ist das Durchführen des Ballons 2, umhüllt vom Schlauch 5 durch eine Düse 6 gezeigt, wobei das Durchführen in der hier gewählten Darstellung von links nach rechts erfolgt. Die Düse 6 hat im Eintrittsbereich 7 einen größeren Querschnitt als im Austrittsbereich 8, d. h. der Schlauch 5 sowie die verschiedenen Schichten des Ballons 2 werden enger zusammengepresst, so dass der vom Schlauch 5 umhüllte Ballon 2 insgesamt nach Austritt aus der Düse 6 einen geringeren Querschnitt aufweist als vorher.

## Patentansprüche

1. Verfahren zur Herstellung eines Katheters (1) mit einem proximalen Abschnitt, einem distalen Abschnitt und einem im distalen Abschnitt angeordneten expandierbaren Element (2), wobei das expandierbare Element (2) durch Zufuhr eines Fluids aus einem kontrahierten Zustand in einen expandierten Zustand bringbar und eine entfernbare Schutzeinrichtung in Form eines Schlauchs (5) um das expandierbare Element (2) im kontrahierten Zustand angeordnet ist, wobei der Schlauch (5) über das expandierbare Element (2) gestülpt wird,
**dadurch gekennzeichnet, dass** der Schlauch (5) mit dem Katheter (1) durch eine oder mehrere Düsen (6) geführt wird, wobei der Schlauch (5) gestreckt wird und sich der Innendurchmesser des Schlauchs (5) verkleinert.

2. Verfahren nach Anspruch 1, **dadurch gekennzeichnet, dass** die Düse (6) die Form eines Hohlzylinders aufweist.

3. Verfahren nach Anspruch 2, **dadurch gekennzeichnet, dass** die Grundfläche des Zylinders ein Kreis oder eine Ellipse ist.

4. Verfahren nach Anspruch 1, **dadurch gekennzeichnet, dass** die Düse (6) eine Öffnung in einem Werkzeug ist.

5. Verfahren nach Anspruch 1, **dadurch gekennzeichnet, dass** die Düse (6) eine Konusform aufweist.

6. Verfahren nach einem der Ansprüche 1 bis 5, **dadurch gekennzeichnet, dass** der Katheter (1) zusammen mit dem Schlauch (5) nacheinander durch mehrere Düsen (6) geführt wird, wobei der Innendurchmesser einer Düse (6) jeweils kleiner ist als der Innendurchmesser der Düse (6), durch die der Katheter (1) zusammen mit dem Schlauch (5) zuvor geführt wurde.

7. Verfahren nach einem der Ansprüche 1 bis 6, **dadurch gekennzeichnet, dass** die Düse (6) beim Durchziehen des Schlauchs (5) erwärmt wird.

8. Verfahren nach einem der Ansprüche 1 bis 7, **dadurch gekennzeichnet, dass** der Schlauch (5) aus Polytetrafluorethylen gefertigt ist.

9. Verfahren nach einem der Ansprüche 1 bis 8, **dadurch gekennzeichnet, dass** das expandierbare Element (2) vor dem Überstülpen des Schlauchs (5) in Falten (4) gelegt wird.

## Claims

1. Method for producing a catheter (1) having a proximal segment, a distal segment, and an expandable element (2) arranged in the distal segment, it being possible to bring the expandable element (2) from a contracted state into an expanded state by supply of a fluid and there being arranged around the expandable element (2) in the contracted state a removable protector in the form of a tube (5), the tube (5) being pulled over the expandable element (2),
**characterized in that** the tube (5) is guided with the catheter (1) through one or more nozzles (6), stretching the tube (5) and reducing the inner diameter of the tube (5).

2. Method according to Claim 1, **characterized in that** the nozzle (6) has the shape of a hollow cylinder.

3. Method according to Claim 2, **characterized in that** the footprint of the cylinder is a circle or an ellipse.

4. Method according to Claim 1, **characterized in that** the nozzle (6) is an opening in a tool.

5. Method according to Claim 1, **characterized in that** the nozzle (6) has a conical shape.

6. Method according to any of Claims 1 to 5,
**characterized in that** the catheter (1) is successively guided together with the tube (5) through multiple nozzles (6), the inner diameter of one nozzle (6) being in each case smaller than the inner diameter of the nozzle (6) through which the catheter (1) was previously guided together with the tube (5).

7. Method according to any of Claims 1 to 6,
**characterized in that** the nozzle (6) is heated during pull-through of the tube (5).

8. Method according to any of Claims 1 to 7,
**characterized in that** the tube (5) is manufactured from polytetrafluoroethylene.

9. Method according to any of Claims 1 to 8,
**characterized in that** the expandable element (2) is laid in folds (4) before the tube (5) is pulled over it.

## Revendications

1. Procédé de fabrication d'un cathéter (1) muni d'une section proximale, d'une section distale et d'un élément expansible (2) agencé dans la section distale, l'élément expansible (2) pouvant être amené par apport d'un fluide à partir d'un état contracté dans un état expansé et un appareil de protection amovible sous la forme d'un tuyau (5) étant agencé autour de l'élément expansible (2) dans l'état contracté, le tuyau (5) étant enfoncé sur l'élément expansible (2),
**caractérisé en ce que**
le tuyau (5) est acheminé avec le cathéter (1) au travers d'une ou de plusieurs buses (6), le tuyau (5) étant étiré et le diamètre intérieur du tuyau (5) se réduisant.

2. Procédé selon la revendication 1, **caractérisé en ce que** la buse (6) présente la forme d'un cylindre creux.

3. Procédé selon la revendication 2, **caractérisé en ce que** la surface de base du cylindre est un cercle ou une ellipse.

4. Procédé selon la revendication 1, **caractérisé en ce que** la buse (6) est une ouverture dans un outil.

5. Procédé selon la revendication 1, **caractérisé en ce que** la buse (6) présente une forme conique.

6. Procédé selon l'une quelconque des revendications 1 à 5, **caractérisé en ce que** le cathéter (1) est acheminé conjointement avec le tuyau (5) successivement au travers de plusieurs buses (6), le diamètre intérieur d'une buse (6) étant à chaque plus petit que le diamètre intérieur de la buse (6) au travers de laquelle le cathéter a été acheminé auparavant conjointement avec le tuyau (5).

7. Procédé selon l'une quelconque des revendications 1 à 6, **caractérisé en ce que** la buse (6) est chauffée lors du passage du tuyau (5).

8. Procédé selon l'une quelconque des revendications 1 à 7, **caractérisé en ce que** le tuyau (5) est fabriqué en polytétrafluoroéthylène.

9. Procédé selon l'une quelconque des revendications 1 à 8, **caractérisé en ce que** l'élément expansible (2) est mis en plis (4) avant l'enfoncement du tuyau (5).
